# EUROPEAN PATENT APPLICATION

(11) **EP 4 593 037 A1**
(43) Date of publication of application: **30.07.2025**
(21) Application number: 23868054.0
(22) Date of filing: 07.09.2023
(51) Int. Cl.: G16H 50/30

(54) **NEURODEGENERATIVE DISEASE RISK DETERMINATION METHOD AND DETERMINATION DEVICE**

(30) Priority: 20.09.2022 JP 2022149488
(71) Applicant: Juntendo Educational Foundation, Tokyo 113-8421 (JP); RIKEN, Wako-shi, Saitama 351-0198 (JP)
(72) Inventor: AKAZAWA, Chihiro, Tokyo 113-8421 (JP); HATTORI, Masahira, Tokyo 113-8421 (JP); HISAMATSU, Daisuke, Tokyo 113-8421 (JP); HATTORI, Nobutaka, Tokyo 113-8421 (JP); SUDA, Wataru, Wako-shi, Saitama 351-0198 (JP); OGATA, Yusuke, Wako-shi, Saitama 351-0198 (JP); ASADA, Takashi, Tokyo 113-0034 (JP)
(74) Representative: Grünecker Patent- und Rechtsanwälte PartG mbB
(86) International application number: PCT/JP2023/032629
(87) International publication number: WO 2024/062931

(57) **Abstract**

Provided is a method and apparatus for risk determination which uses an easily obtainable saliva sample to estimate risks of neurodegenerative diseases through stratification which differentiates multiple diseases. The invention provides a method of risk determination which estimates risks of neurodegenerative diseases through stratification which differentiates multiple diseases, the method executed by a computer processor, comprising a step of acquiring representation abundances of multiple types of bacteria from an analysis of salivary microbiota of a test subject; and a step of inputting the acquired representation abundances into a prediction model to estimate risks of neurodegenerative diseases occurring in the test subject through stratification which differentiates multiple diseases, wherein the prediction model has been generated by machine learning which entails obtaining an algorithm using, as explanatory variables, representation abundances of multiple types of bacteria acquired from an analysis of salivary microbiota of healthy subjects and patients of multiple diseases belonging to the neurodegenerative diseases, and the disease states of the healthy subjects and the patients stratified across the multiple diseases, output as target variables, as training data, wherein said multiple types of bacteria include bacteria types having high representation abundances and/or bacteria types showing significant differences in the representation abundances between patients of different diseases in the analysis of the salivary microbiota of the patients stratified.

## Description

### Technical Field

The present invention relates to methods and apparatuses for determining risks of neurodegenerative diseases.

### Background Art

Neurodegenerative diseases are a group of central nervous system diseases each of which results from disorder/disintegration of a characteristic group of neurons. Clinically, neurodegenerative diseases refer to the diseases with unknown causes which have latent onset and show slowly progressive mental/neural symptoms. In recent years, the neurodegenerative diseases have been classified according to main aberrant proteins accumulating in the neurons or glial cells, or their patterns of accumulation, based on the concept of proteinopathy which postulates that a same pathogenic protein can cause a common pathological state. The classifications include tauopathies such as Alzheimer's disease (AD), TDP-43 proteinopathies such as amyotrophic lateral sclerosis (ALS), and synucleinopathies such as Parkinson's disease (PD), dementia with Lewy bodies (DLB), and multiple system atrophy (MSA).

There is still no fundamental therapeutic drug for the neurodegenerative diseases. Often, these neurodegenerative diseases have already progressed to pathologically intractable stages when clinical symptoms become evident. Therefore, there is a need for the development of methods for early diagnosis and for determining risks of the neurodegenerative diseases, as well as early stratification of their underlying diseases.

There are recent reports that microbiota (microbiomes) inhabiting the gut or oral cavity are closely involved in the formation of physiological states and disease states including those of the host immune system. Even in dementia such as Alzheimer's disease, it is becoming clear that there is a correlation between decline of cognitive functions and changes in the microbiota. It is suggested that microbiomes have a potential to provide biomarkers for the neurodegenerative diseases. There is a report that a logistic regression analysis based on the profiling of salivary microbiomes was used to construct a prediction model using several microbial species which could distinguish between mild cognitive impairment (MCI) and Alzheimer's disease (Non-Patent Document 1). There is also a report that a logistic regression analysis based on the microbiome genus and species data obtained by sequencing the salivary RNAs could distinguish between early-stage Parkinson's disease (PD) patients and healthy subjects using the data on 11 species of bacteria (Non-Patent Document 2).

### Citation List

### Non-Patent Documents

Non-Patent Document 1: Alzheimer's Dementia, 2020, vol. 12, e12000
Non-Patent Document 2: PlosOne 2019 vol. 14, No. 6, e0218252

### Summary of the Invention

### Problem to Be Solved by the Invention

However, stratification to differentiate healthy subjects and multiple diseases was not obtained by any of these means, and a biomarker for stratifying various neurodegenerative diseases has not been developed.

Thus, an objective of the present invention is the provision of a method of risk determination which uses an easily obtainable saliva sample and by which risks of the neurodegenerative diseases can be estimated through stratification which differentiates multiple diseases.

### Solution to the Problem

The present inventors discovered that risks of neurodegenerative diseases occurring in a test subject can be estimated through stratification which differentiates multiple diseases, by using a prediction model generated by machine learning which entails obtaining an algorithm using, as explanatory variables, representation abundances of multiple types of bacteria acquired from an analysis of salivary microbiota of healthy subjects and patients of multiple diseases belonging to the neurodegenerative diseases, and the disease states of the healthy subjects and the patients stratified across the multiple diseases belonging to neurodegenerative diseases, output as target variables, as training data; and applying representation abundances of the multiple types of bacteria acquired from an analysis of salivary microbiota of the test subject into the prediction model. The discovery led to the completion of the invention.

Accordingly, the present invention provides the following inventions [1] to [8].
[1] A method of risk determination which estimates risks of neurodegenerative diseases through stratification which differentiates multiple diseases, the method executed by a processor of a computer, wherein the method comprises
   a step of acquiring representation abundances of multiple types of bacteria from an analysis of salivary microbiota of a test subject; and
   a step of inputting the acquired representation abundances of multiple types of bacteria into a prediction model to estimate risks of neurodegenerative diseases occurring in the test subject through stratification which differentiates multiple diseases,
   wherein the prediction model has been generated by machine learning which entails obtaining an algorithm using, as explanatory variables, representation abundances of the multiple types of bacteria acquired from an analysis of salivary microbiota of healthy subjects and patients of multiple diseases belonging to the neurodegenerative diseases, and the disease states of the healthy subjects and the patients stratified across the multiple diseases belonging to neurodegenerative diseases, output as target variables, as training data,
   wherein said multiple types of bacteria include bacteria types having high representation abundances in the analysis of the salivary microbiota of the patients stratified, and/or bacteria types showing significant differences in the representation abundances between patients of different diseases in the analysis of the salivary microbiota of the patients stratified.
[2] The method of risk determination according to [1], wherein the stratification which differentiates multiple diseases comprises stratification to differentiate healthy aged subjects, mild cognitive impairment (MCI), and dementia (DE), stratification to differentiate healthy aged subjects, mild cognitive impairment (MCI), dementia (DE), and dementia with Lewy bodies (DLB), or stratification to differentiate Parkinson's disease (PD) and dementia with Lewy bodies (DLB).
[3] The method of risk determination according to [1], wherein the multiple diseases include dementia with Lewy bodies (DLB) and Parkinson's disease (PD).
[4] The method of risk determination according to any of [1] to [3], wherein the multiple types of bacteria include one or more types of bacteria selected from the group consisting of [Eubacterium] brachy, Porphyromonas endodontalis, Alloprevotella tannerae, Capnocytophaga leadbetteri, Streptococcus gordonii, Campylobacter concisus, Tannerella forsythia, Filifactor alocis, [Eubacterium] nodatum, Streptococcus cristatus, Neisseria elongata, Treponema denticola, Actinomyces oris, [Eubacterium] saphenum, Streptococcus constellatus, Parvimonas micra, Prevotella denticola, Leptotrichia hofstadii, Fusobacterium nucleatum, Catonella morbi, Lactobacillus antri, Alloprevotella rava, Streptococcus anginosus, Prevotella jejuni, Streptococcus mitis, Gemella haemolysans, Neisseria macacae, Prevotella multiformis, Abiotrophia defectiva, Streptococcus salivarius, Streptococcus lactarius, Corynebacterium matruchotii, Oribacterium asaccharolyticum, Prevotella loescheii, Aggregatibacter segnis, Peptostreptococcus stomatis, Veillonella infantium, Capnocytophaga granulosa, Leptotrichia buccalis, Veillonella atypica, Streptococcus pseudopneumoniae, Corynebacterium durum, Granulicatella adiacens, [Eubacterium] sulci, Selenomonas infelix, Capnocytophaga sputigena, Lactobacillus crispatus, Streptococcus parasanguinis, Rothia mucilaginosa, Streptococcus sobrinus, Atopobium parvulum, Solobacterium moorei, Neisseria perflava, Bifidobacterium dentium, Actinomyces graevenitzii, Streptococcus mutans, Prevotella pallens, Porphyromonas gingivalis, Rothia dentocariosa, Fusobacterium periodonticum, Lactobacillus fermentum, Prevotella melaninogenica, Leptotrichia wadei, Lautropia mirabilis, Streptococcus infantis, Neisseria oralis, Prevotella pleuritidis, Prevotella oris, Lactobacillus paracasei, Lachnoanaerobaculum orale, Haemophilus parahaemolyticus, Prevotella nanceiensis, Lactobacillus salivarius, Streptococcus sanguinis, Haemophilus parainfluenzae, Lactobacillus vaginalis, Bacteroides heparinolyticus, Prevotella salivae, Gemella morbillorum, Gemella sanguinis, Prevotella shahii, Haemophilus haemolyticus, Schaalia odontolytica, Lactobacillus gasseri, Streptococcus australis, Streptococcus oralis, Prevotella histicola, Schaalia meyeri, Porphyromonas pasteri, Prevotella intermedia, Granulicatella elegans, Streptococcus downei, Parascardovia denticolens, Staphylococcus aureus, Haemophilus sputorum, and Prevotella oulorum.
[5] An apparatus for risk determination which estimates risks of neurodegenerative diseases through stratification which differentiates multiple diseases, the apparatus comprising
   a processor,
   a storage unit which stores a computer program executed by the processor, and
   a communication circuit which receives representation abundances of multiple types of bacteria from an analysis of microbiota of saliva obtained from a test subject;
   wherein the processor executes the computer program to acquire the representation abundances of multiple types of bacteria received by the communication circuit and inputs the acquired representation abundances of multiple types of bacteria into a prediction model to estimate risks of neurodegenerative diseases occurring in the test subject through stratification which differentiates multiple diseases,
   wherein the prediction model has been generated by machine learning which entails obtaining an algorithm using, as explanatory variables, representation abundances of the multiple types of bacteria acquired from an analysis of salivary microbiota of healthy subjects and patients of multiple diseases belonging to the neurodegenerative diseases, and the disease states of the healthy subjects and the patients stratified across the multiple diseases belonging to neurodegenerative diseases, output as target variables, as training data,
   wherein said multiple types of bacteria include bacteria types having high representation abundances in the analysis of the salivary microbiota of the patients stratified, and/or bacteria types showing significant differences in the representation abundances between patients of different diseases in the analysis of the salivary microbiota of the patients stratified.
[6] The apparatus for risk determination according to [5], wherein the stratification which differentiates multiple diseases comprises stratification to differentiate healthy aged subjects, mild cognitive impairment (MCI), and dementia (DE), stratification to differentiate healthy aged subjects, mild cognitive impairment (MCI), dementia (DE), and dementia with Lewy bodies (DLB), or stratification to differentiate Parkinson's disease (PD) and dementia with Lewy bodies (DLB).
[7] The apparatus for risk determination according to [5], wherein the multiple diseases include dementia with Lewy bodies (DLB) and Parkinson's disease (PD).
[8] The apparatus for risk determination according to any of [5] to [7], wherein the multiple types of bacteria include one or more types of bacteria selected from the group consisting of [Eubacterium] brachy, Porphyromonas endodontalis, Alloprevotella tannerae, Capnocytophaga leadbetteri, Streptococcus gordonii, Campylobacter concisus, Tannerella forsythia, Filifactor alocis, [Eubacterium] nodatum, Streptococcus cristatus, Neisseria elongata, Treponema denticola, Actinomyces oris, [Eubacterium] saphenum, Streptococcus constellatus, Parvimonas micra, Prevotella denticola, Leptotrichia hofstadii, Fusobacterium nucleatum, Catonella morbi, Lactobacillus antri, Alloprevotella rava, Streptococcus anginosus, Prevotella jejuni, Streptococcus mitis, Gemella haemolysans, Neisseria macacae, Prevotella multiformis, Abiotrophia defectiva, Streptococcus salivarius, Streptococcus lactarius, Corynebacterium matruchotii, Oribacterium asaccharolyticum, Prevotella loescheii, Aggregatibacter segnis, Peptostreptococcus stomatis, Veillonella infantium, Capnocytophaga granulosa, Leptotrichia buccalis, Veillonella atypica, Streptococcus pseudopneumoniae, Corynebacterium durum, Granulicatella adiacens, [Eubacterium] sulci, Selenomonas infelix, Capnocytophaga sputigena, Lactobacillus crispatus, Streptococcus parasanguinis, Rothia mucilaginosa, Streptococcus sobrinus, Atopobium parvulum, Solobacterium moorei, Neisseria perflava, Bifidobacterium dentium, Actinomyces graevenitzii, Streptococcus mutans, Prevotella pallens, Porphyromonas gingivalis, Rothia dentocariosa, Fusobacterium periodonticum, Lactobacillus fermentum, Prevotella melaninogenica, Leptotrichia wadei, Lautropia mirabilis, Streptococcus infantis, Neisseria oralis, Prevotella pleuritidis, Prevotella oris, Lactobacillus paracasei, Lachnoanaerobaculum orale, Haemophilus parahaemolyticus, Prevotella nanceiensis, Lactobacillus salivarius, Streptococcus sanguinis, Haemophilus parainfluenzae, Lactobacillus vaginalis, Bacteroides heparinolyticus, Prevotella salivae, Gemella morbillorum, Gemella sanguinis, Prevotella shahii, Haemophilus haemolyticus, Schaalia odontolytica, Lactobacillus gasseri, Streptococcus australis, Streptococcus oralis, Prevotella histicola, Schaalia meyeri, Porphyromonas pasteri, Prevotella intermedia, Granulicatella elegans, Streptococcus downei, Parascardovia denticolens, Staphylococcus aureus, Haemophilus sputorum, and Prevotella oulorum.

### Effect of the Invention

According to the methods and apparatuses of the invention, an easily obtainable, small amount of saliva sample can be used to perform accurate risk determination of neurodegenerative diseases through stratification which differentiates multiple diseases, such as stratification to differentiate healthy subjects, MCI, and dementia, or stratification to differentiate proteinopathy classes such as AD and DLB, or DLB and PD. Therefore, the inventive methods and apparatuses may be useful as a means for determining risks of neurodegenerative diseases in regular physical examinations and may contribute to early diagnosis of neurodegenerative diseases in a stratified manner.

### Brief Descriptions of the Figures

[Figure 1] The figure illustrates constitution of a determination method according to the invention.
[Figure 2] The figure exemplifies constitution of a risk determination apparatus.
[Figure 3] The figure provides a flowchart exemplifying the operation of the determination process in a determination method and a risk determination apparatus according to the invention.
[Figure 4] The figure provides a flowchart exemplifying the operation of the training process in a determination method and a risk determination apparatus according to the invention.
[Figure 5] The figure shows AUC-RFs obtained from a prediction model which was built by performing machine learning based on representation abundances of the 94 types of bacteria having the high representation abundances at the species level (Table 1).
[Figure 6] The figure shows AUC-RFs obtained from a prediction model which was built by performing machine learning based on representation abundances of the 95 types of bacteria having the high representation abundances at the species level (Table 2).
[Figure 7] The figure shows AUC-RFs obtained from a prediction model which was built by performing machine learning based on representation abundances of the 34 types of bacteria having the high representation abundances at the species level (Table 3).
[Figure 8] The figure shows AUC-RFs obtained from a prediction model which was built by performing machine learning based on representation abundances of the 74 types of bacteria having the high representation abundances at the species level (Table 4).

### Detailed Description of the Invention

The present invention provides risk determination methods and determination apparatuses which estimate risks of neurodegenerative diseases through stratification which differentiates multiple diseases.

In the present specification, "neurodegenerative diseases" refer to a group of diseases, as described above, each of which has characteristic regions of the nervous system afflicted and exhibits various degenerative changes mainly in neurons. Specific examples include tauopathies such as Alzheimer's disease (AD); TDP-43 proteinopathies such as amyotrophic lateral sclerosis (ALS); and synucleinopathies such as Parkinson's disease (PD), dementia with Lewy bodies (DLB), and multiple system atrophy (MSA). The neurodegenerative diseases in the present invention also include mild cognitive impairment (MCI). In the present specification, dementia (DE) unless otherwise specified collectively refers to tauopathies showing cognitive decline, including AD.

Besides Alzheimer's disease, examples of tauopathies include primary age-related tauopathy, chronic traumatic encephalopathy, progressive supranuclear palsy, corticobasal degeneration, FTDP-17, and lytico-bodig disease.

In the present specification, stratification means differentiating into groups. Thus, in the present invention, any plural number of diseases among these neurodegenerative diseases can be assessed by stratification. Examples include stratification to differentiate healthy aged subjects, MCI, and dementia (DE), stratification to differentiate healthy aged subjects, MCI, dementia (DE), and DLB, and stratification to differentiate PD and DLB.

Stratification to differentiate healthy subjects, MCI, dementia, and DLB, or stratification to differentiate PD and DLB is more preferable. The ability of stratification to differentiate PD and DLB, despite the fact that these diseases are both synucleinopathies, is extremely useful. Stratification of dementia (DE) into tauopathies and synucleinopathies is also possible.

"Saliva" refers to the secretion liquid normally secreted from salivary glands into the oral cavity. The saliva subjected to the surveying in the present invention is saliva taken from healthy subjects and patients of neurodegenerative diseases.

"Salivary microbiota" means a population of live bacteria in the saliva, and its genetic information may be referred to as microbiome. In the present invention, salivary microbiota is preferably handled in the form of microbiome, as it is preferably determined by surveying genetic information of the bacteria, particularly that of 16S rRNA.

An embodiment of the inventive method of risk determination is a method of risk determination which estimates risks of neurodegenerative diseases through stratification which differentiates multiple diseases, the method executed by a processor of a computer, wherein the method comprises
a step of acquiring representation abundances of multiple types of bacteria from an analysis of salivary microbiota of a test subject; and
a step of inputting the acquired representation abundances of multiple types of bacteria into a prediction model to estimate risks of neurodegenerative diseases occurring in the test subject through stratification which differentiates multiple diseases,
wherein the prediction model has been generated by machine learning which entails obtaining an algorithm using, as explanatory variables, representation abundances of the multiple types of bacteria acquired from an analysis of salivary microbiota of healthy subjects and patients of multiple diseases belonging to the neurodegenerative diseases, and the disease states of the healthy subjects and the patients stratified across the multiple diseases belonging to neurodegenerative diseases, output as target variables, as training data,
wherein said multiple types of bacteria include bacteria types having high representation abundances in the analysis of the salivary microbiota of the patients stratified, and/or bacteria types showing significant differences in the representation abundances between patients of different diseases in the analysis of the salivary microbiota of the patients stratified.

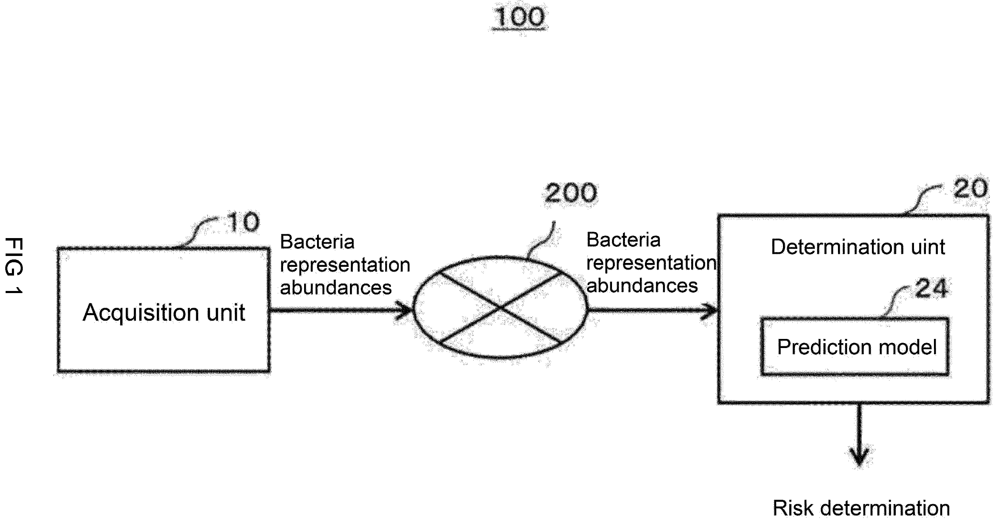

The inventive method as shown in Figure 1 may comprise Step 1 in which bacterial representation abundance acquisition unit 10 acquires representation abundances of multiple types of bacteria from an analysis of salivary microbiota of a test subject; and Step 2 in which determination unit 20 in the processor of the computer inputs the acquired representation abundances of multiple types of bacteria into a prediction model to estimate risks of neurodegenerative diseases occurring in the test subject through stratification which differentiates multiple diseases.

Here, Step 1 can be carried out for example as follows.

For the bacteria contained in a saliva sample, the V1-V2 region of 16S rRNA is amplified by PCR, and nucleobase sequences of the fragments amplified by PCR are determined by using a next generation sequencer. Next, among the determined nucleobase sequences (reads), those that have passed quality checks are subjected to clustering according to 97% similarity to provide Operational Taxonomic Units (OTUs). 16S rRNA nucleobase sequences deposited in a genome database are referenced against the nucleobase sequences of the OTUs to identify bacteria type of each OTU. From the read count of each OTU, representation abundance of each bacteria type can be identified.

The step of acquiring representation abundances of multiple types of bacteria from an analysis of salivary microbiota for building a prediction model may also be done in the same way.

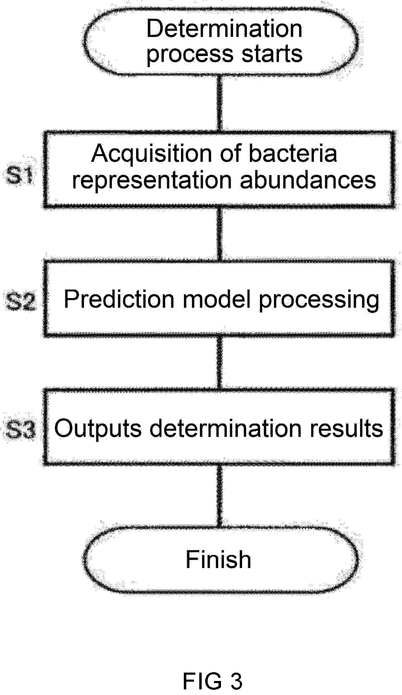

Step 2 may be carried out by inputting the acquired representation abundances of multiple types of bacteria into a prediction model to estimate risks of neurodegenerative diseases occurring in the test subject through stratification which differentiates multiple diseases, which is carried out by determination unit 20 in the processor of the computer. The prediction model has been input, in advance, to the processor of the computer performing Step 2 and thus, as shown in Figure 3, when the representation abundances of multiple types of bacteria acquired in Step 1 are input into the prediction model, risks of neurodegenerative diseases occurring in the test subject can be estimated through stratification which differentiates multiple diseases, to output a prediction result.

Next, building of a prediction model is described.

The prediction model is generated by machine learning which entails obtaining an algorithm using, as explanatory variables, representation abundances of multiple types of bacteria acquired from an analysis of salivary microbiota of healthy subjects and patients of multiple diseases belonging to neurodegenerative diseases, and the disease states of the healthy subjects and the patients stratified across the multiple diseases belonging to neurodegenerative diseases, output as target variables, as training data.

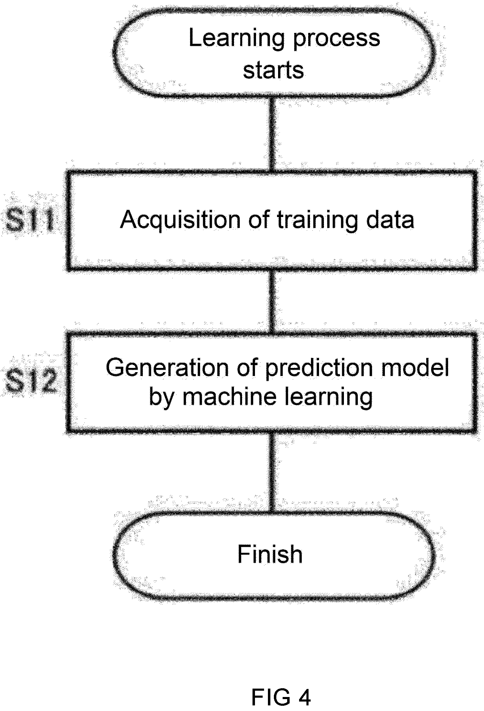

For example, as shown in Figure 4, representation abundances of multiple types of bacteria acquired from an analysis of salivary microbiota of healthy subjects and patients of multiple diseases belonging to the neurodegenerative diseases, which are explanatory variables, are acquired, and using these, a prediction model may be generated by machine learning which entails an algorithm obtained for target variables, which is for outputting the disease states of the healthy subjects and the patients stratified across the multiple diseases belonging to neurodegenerative diseases, as training data.

More specifically, for example, the bacteria types and their representation abundances are analyzed on a cohort consisting of healthy aged controls (HC), patients of mild cognitive impairment (MCI), and dementia (DE). From the analysis of the bacteria types and their representation abundances, those bacteria types having high representation abundances and/or characteristic bacteria types (bacteria types showing significant differences to serve as markers) are specified. Then, a prediction model can be built by machine learning which entails obtaining an algorithm using, as explanatory variables, representation abundances of multiple types of bacteria acquired from an analysis of salivary microbiota of healthy subjects and patients of multiple diseases belonging to the neurodegenerative diseases, and the disease states of the healthy subjects and the patients stratified across the multiple diseases belonging to neurodegenerative diseases, output as target variables, as training data.

The machine learning herein can for example employ random forest.

In the present invention, stratification can be achieved wherein the multiple types of bacteria include bacteria types having high representation abundances in the analysis of the salivary microbiota of the patients stratified, and/or bacteria types showing significant differences in the representation abundances between patients of different diseases in the analysis of the salivary microbiota of the patients stratified, thereby risks of neurodegenerative diseases can be estimated through stratification which differentiates multiple diseases.

More specifically, stratification to differentiate healthy subjects, MCI, dementia, and DLB, stratification to differentiate PD and DLB, etc., can be performed. Stratification to differentiate healthy subjects, MCI, dementia, and DLB and stratification to differentiate PD and DLB are more preferable. The ability of stratification to differentiate these diseases, despite the fact that PD and DLB are both synucleinopathies, is extremely useful.

PD can be classified according to the severity. Herein, PD is classified based on the Hoehn & Yahr severity scales, into mild (Hoehn & Yahr severity scale 1 or 2) and severe (Hoehn & Yahr severity scale 3 or higher). More specifically, stratification can be performed to differentiate healthy subjects, DLB, mild PD, and severe PD. Or, PD is classified based on the duration of the disease into early stage (5 years or less since the onset) and late stage (6 years or more). Stratification to differentiate healthy subjects, DLB, early stage PD and late stage PD can be performed.

Examples of bacteria that can be used for risk determination of neurodegenerative diseases through stratification across multiple diseases include one or two or more of the bacteria shown in Table 1 below. The bacteria types shown in Table 1 are those which have been used for stratification to differentiate healthy subjects, MCI, DE, and DLB.

**[Table 1]**

| |
|---|
| [Eubacterium] brachy |
| Porphyromonas endodontalis |
| Alloprevotella tannerae |
| Capnocytophaga leadbetteri |
| Streptococcus gordonii |
| Campylobacter concisus |
| Tannerella forsythia |
| Filifactor alocis |
| [Eubacterium] nodatum |
| Streptococcus cristatus |
| Neisseria elongate |
| Treponema denticola |
| Actinomyces oris |
| [Eubacterium] saphenum |
| Streptococcus constellatus |
| Parvimonas micra |
| Prevotella denticola |
| Leptotrichia hofstadii |
| Fusobacterium nucleatum |
| Catonella morbi |
| Lactobacillus antri |
| Alloprevotella rava |
| Streptococcus anginosus |
| Prevotella jejuni |
| Streptococcus mitis |
| Gemella haemolysans |
| Neisseria macacae |
| Prevotella multiformis |
| Abiotrophia defectiva |
| Streptococcus salivarius |
| Streptococcus lactarius |
| Corynebacterium matruchotii |
| Oribacterium asaccharolyticum |
| Prevotella loescheii |
| Aggregatibacter segnis |
| Peptostreptococcus stomatis |
| Veillonella infantium |
| Capnocytophaga granulosa |
| Leptotrichia buccalis |
| Veillonella atypica |
| Streptococcus pseudopneumoniae |
| Corynebacterium durum |
| Granulicatella adiacens |
| [Eubacterium] sulci |
| Selenomonas infelix |
| Capnocytophaga sputigena |
| Lactobacillus crispatus |
| Streptococcus parasanguinis |
| Rothia mucilaginosa |
| Streptococcus sobrinus |
| Atopobium parvulum |
| Solobacterium moorei |
| Neisseria perflava |
| Bifidobacterium dentium |
| Actinomyces graevenitzii |
| Streptococcus mutans |
| Prevotella pallens |
| Porphyromonas gingivalis |
| Rothia dentocariosa |
| Fusobacterium periodonticum |
| Lactobacillus fermentum |
| Prevotella melaninogenica |
| Leptotrichia wadei |
| Lautropia mirabilis |
| Streptococcus infantis |
| Neisseria oralis |
| Prevotella pleuritidis |
| Prevotella oris |
| Lactobacillus paracasei |
| Lachnoanaerobaculum orale |
| Haemophilus parahaemolyticus |
| Prevotella nanceiensis |
| Lactobacillus salivarius |
| Streptococcus sanguinis |
| Haemophilus parainfluenzae |
| Lactobacillus vaginalis |
| Bacteroides heparinolyticus |
| Prevotella salivae |
| Gemella morbillorum |
| Gemella sanguinis |
| Prevotella shahii |
| Haemophilus haemolyticus |
| Schaalia odontolytica |
| Lactobacillus gasseri |
| Streptococcus australis |
| Streptococcus oralis |
| Prevotella histicola |
| Schaalia meyeri |
| Porphyromonas pasteri |
| Prevotella intermedia |
| Granulicatella elegans |
| Streptococcus downei |
| Parascardovia denticolens |

The bacteria types used for healthy subjects, DLB, and PD may comprise one or two or more of bacteria types selected from the bacteria types shown in Table 2.

**[Table 2]**

| |
|---|
| Streptococcus salivarius |
| Streptococcus mitis |
| Rothia mucilaginosa |
| Streptococcus parasanguinis |
| Neisseria mucosa |
| Prevotella melaninogenica |
| Veillonella atypica |
| Abiotrophia para-adiacens |
| Streptococcus infantis |
| Streptococcus australis |
| Prevotella histicola |
| Haemophilus parainfluenzae |
| Gemella haemolysans |
| Streptococcus cristatus |
| Gemella sanguinis |
| Actinomyces sp. oral taxon 180 |
| Fusobacterium periodonticum |
| Prevotella pallens |
| Porphyromonas pasteri |
| Leptotrichia genomosp. C1 |
| Neisseria macacae |
| Streptococcus sanguinis |
| Prevotella sp. oral taxon 306 |
| Actinomyces graevenitzii |
| Streptococcus oralis |
| Fusobacterium nucleatum |
| Prevotella sp. oral clone GI032 |
| Streptococcus gordonii |
| Prevotella sp. oral clone DO039 |
| Prevotella jejuni |
| Okadaella gastrococcus |
| Rothia sp. Smarlab 3302411 |
| Streptococcus sp. oral taxon 058 |
| Oribacterium sp. OBRC12 |
| Streptococcus sp. C300 |
| Lachnoanaerobaculum sp. OBRC5-5 |
| Abiotrophia defectiva |
| Leptotrichia sp. oral clone FP036 |
| Porphyromonas gingivalis |
| Solobacterium moorei |
| Lautropia mirabilis |
| Neisseria oralis |
| Alloprevotella tannerae |
| Leptotrichia sp. oral clone DR011 |
| Rothia dentocariosa |
| [Eubacterium] sulci |
| Alloprevotella rava |
| Streptococcus sp. oral strain T1-E5 |
| Peptostreptococcus stomatis |
| Prevotella nanceiensis |
| Porphyromonas endodontalis |
| Campylobacter concisus |
| Parvimonas micra |
| uncultured Prevotellaceae bacterium |
| Streptococcus anginosus |
| Prevotella oris |
| Prevotella denticola |
| Streptococcus genomosp. C8 |
| Streptococcus sp. 34583 |
| Prevotella intermedia |
| Lactobacillus fermentum |
| Granulicatella elegans |
| Atopobium sp. ICM42b10 |
| Lactobacillus salivarius |
| Eubacterium sp. oral clone DO008 |
| Porphyromonas sp. oral clone BS045 |
| Neisseria elongata |
| Actinomyces sp. ph3 |
| uncultured Prevotella sp. |
| Corynebacterium matruchotii |
| Gemella morbillorum |
| Capnocytophaga granulosa |
| Streptococcus constellatus |
| Porphyromonas sp. oral clone EP003 |
| Capnocytophaga leadbetteri |
| Catonella sp. oral clone EZ006 |
| Leptotrichia sp. |
| Streptococcus genomosp. C6 |
| Schaalia odontolytica |
| Actinomyces sp. ICM47 |
| Lactobacillus gasseri |
| Filifactor alocis |
| Streptococcus mutans |
| Streptococcus sobrinus |
| Aggregatibacter segnis |
| uncultured Eubacterium sp. |
| Prevotella sp. oral clone FM005 |
| Haemophilus sp. COAD165 |
| uncultured Corynebacterium sp. |
| Streptococcus downei |
| Megasphaera micronuciformis |
| TM7 phylum sp. oral clone DR034 |
| Lactobacillus crispatus |
| Actinomyces sp. ICM58 |
| Staphylococcus aureus |

The bacteria types used for healthy subjects, DLB, and PDs (stratified according to severity) may comprise one or two or more types selected from the bacteria types shown in Table 3.

**[Table 3]**

| |
|---|
| Lactobacillus gasseri |
| Streptococcus lactarius |
| Streptococcus oralis |
| Schaalia meyeri |
| Porphyromonas pasteri |
| Streptococcus salivarius |
| Schaalia odontolytica |
| Haemophilus haemolyticus |
| Actinomyces graevenitzii |
| Rothia dentocariosa |
| Neisseria perflava |
| Streptococcus australis |
| Streptococcus pseudopneumoniae |
| Atopobium parvulum |
| Streptococcus sobrinus |
| Capnocytophaga granulosa |
| Capnocytophaga sputigena |
| Veillonella infantium |
| [Eubacterium] brachy |
| Rothia mucilaginosa |
| Fusobacterium periodonticum |
| Prevotella histicola |
| Solobacterium moorei |
| Oribacterium asaccharolyticum |
| Streptococcus cristatus |
| Prevotella salivae |
| Streptococcus infantis |
| Parvimonas micra |
| Streptococcus anginosus |
| Prevotella oulorum |
| Haemophilus sputorum |
| Veillonella atypica |
| Lactobacillus salivarius |
| Alloprevotella tannerae |

The bacteria types used for healthy subjects, DLB, and PDs (stratified according to duration of disease) may comprise one or two or more types selected from Table 4.

**[Table 4]**

| |
|---|
| Rothia mucilaginosa |
| Lautropia mirabilis |
| Lactobacillus gasseri |
| Prevotella denticola |
| Actinomyces graevenitzii |
| Veillonella infantium |
| Lachnoanaerobaculum orale |
| Solobacterium moorei |
| Streptococcus australis |
| Campylobacter concisus |
| Alloprevotella tannerae |
| Haemophilus sputorum |
| [Eubacterium] brachy |
| Neisseria perflava |
| Porphyromonas endodontalis |
| Prevotella oris |
| Streptococcus anginosus |
| Oribacterium asaccharolyticum |
| Lactobacillus crispatus |
| Streptococcus infantis |
| Leptotrichia wadei |
| Streptococcus mutans |
| Streptococcus lactarius |
| Haemophilus haemolyticus |
| Prevotella pallens |
| Streptococcus constellatus |
| Prevotella melaninogenica |
| Capnocytophaga sputigena |
| Veillonella atypica |
| Streptococcus salivarius |
| Streptococcus sanguinis |
| Alloprevotella rava |
| Lactobacillus salivarius |
| Capnocytophaga granulosa |
| Prevotella salivae |
| Catonella morbi |
| [Eubacterium] sulci |
| Schaalia odontolytica |
| Aggregatibacter segnis |
| Fusobacterium periodonticum |
| Rothia dentocariosa |
| Streptococcus gordonii |
| Streptococcus downei |
| Atopobium parvulum |
| Fusobacterium nucleatum |
| Haemophilus parainfluenzae |
| Schaalia meyeri |
| Prevotella jejuni |
| Prevotella histicola |
| Prevotella nanceiensis |
| Neisseria elongata |
| Lactobacillus fermentum |
| Streptococcus cristatus |
| Porphyromonas pasteri |
| Parvimonas micra |
| Gemella sanguinis |
| Streptococcus pseudopneumoniae |
| Prevotella shahii |
| Granulicatella adiacens |
| Peptostreptococcus stomatis |
| Streptococcus oralis |
| Granulicatella elegans |
| Abiotrophia defectiva |
| Gemella haemolysans |
| Undefined |
| Porphyromonas gingivalis |
| Staphylococcus aureus |
| Gemella morbillorum |
| Prevotella intermedia |
| Neisseria macacae |
| Streptococcus parasanguinis |
| Corynebacterium matruchotii |
| Neisseria oralis |
| Streptococcus mitis |

An embodiment of the inventive apparatus for determination is an apparatus for risk determination which estimates risks of neurodegenerative diseases through stratification which differentiates multiple diseases, the apparatus comprising
a processor,
a storage unit which stores a computer program executed by the processor, and
a communication circuit which receives representation abundances of multiple types of bacteria from an analysis of microbiota of saliva obtained from a test subject;
wherein the processor executes the computer program to acquire the representation abundances of multiple types of bacteria received by the communication circuit and inputs the acquired representation abundances of multiple types of bacteria into a prediction model to estimate risks of neurodegenerative diseases occurring in the test subject through stratification which differentiates multiple diseases,
wherein the prediction model has been generated by machine learning which entails obtaining an algorithm using, as explanatory variables, representation abundances of the multiple types of bacteria acquired from an analysis of salivary microbiota of healthy subjects and patients of multiple diseases belonging to the neurodegenerative diseases, and the disease states of the healthy subjects and the patients stratified across the multiple diseases belonging to neurodegenerative diseases, output as target variables, as training data,
wherein said multiple types of bacteria include bacteria types having high representation abundances in the analysis of the salivary microbiota of the patients stratified, and/or bacteria types showing significant differences in the representation abundances between patients of different diseases in the analysis of the salivary microbiota of the patients stratified.

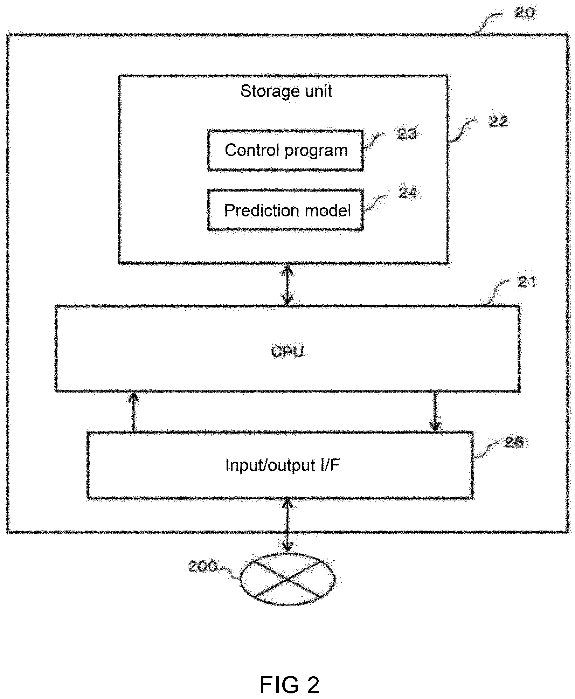

An outline of an apparatus for determination of the invention is shown in Figure 2. Determination unit 20 is comprised of an information processing device such as a computer. Determination unit 20 comprises CPU 21 which performs computation, storage unit 22 which stores various data and a computer program, and an input/output interface (I/F) 26 for conducting communication with other apparatuses.

Prediction model 21 is generated by machine learning which entails obtaining an algorithm using, as explanatory variables, representation abundances of the multiple types of bacteria acquired from an analysis of salivary microbiota of healthy subjects and patients of multiple diseases belonging to neurodegenerative diseases, and the disease states of the healthy subjects and the patients stratified across the multiple diseases belonging to neurodegenerative diseases, output as target variables, as training data.

For example, as shown in Figure 4, representation abundances of the multiple types of bacteria acquired from an analysis of salivary microbiota of healthy subjects and patients of multiple diseases belonging to the neurodegenerative diseases are acquired, which provide explanatory variables, and using those, machine learning with training data can be performed for an algorithm which outputs the disease states of the healthy subjects and the patients stratified across the multiple diseases belonging to neurodegenerative diseases provided as target variables, thereby generating a prediction model.

More specifically, for example, the bacteria types and their representation abundances are analyzed on a cohort consisting of healthy aged controls (HC), patients of mild cognitive impairment (MCI), and patients of dementia (DE). From the analysis of the bacteria types and their representation abundances, those bacteria types having high representation abundances and/or characteristic bacteria types (bacteria types showing significant differences to serve as markers) are specified. Then, a prediction model can be built by machine learning which entails obtaining an algorithm using, as explanatory variables, representation abundances of the multiple types of bacteria acquired from an analysis of salivary microbiota of healthy subjects and patients of multiple diseases belonging to the neurodegenerative diseases, and the disease states of the healthy subjects and the patients stratified across the multiple diseases belonging to neurodegenerative diseases, output as target variables, as training data.

The machine learning herein can for example employ random forest.

In the present invention, stratification can be achieved wherein the multiple types of bacteria include bacteria types having high representation abundances in the analysis of the salivary microbiota of the patients stratified, and/or bacteria types showing significant differences in the representation abundances between patients of different diseases in the analysis of the salivary microbiota of the patients stratified, thereby risks of neurodegenerative diseases can be estimated through stratification which differentiates multiple diseases.

More specifically, stratification to differentiate healthy subjects, MCI, dementia, and DLB, stratification to differentiate PD and DLB, etc., can be performed. Stratification to differentiate healthy subjects, MCI, dementia, and DLB and stratification to differentiate PD and DLB are more preferable. The ability of stratification to differentiate these diseases, despite the fact that PD and DLB are both synucleinopathies, is extremely useful.

To determine risks of neurodegenerative diseases using the inventive determination apparatus, the computer program is executed at the processor to acquire the representation abundances of multiple types of bacteria received by the communication circuit (Step 1) and
the acquired representation abundances of multiple types of bacteria are input into prediction model 21 to estimate risks of neurodegenerative diseases occurring in the test subject through stratification which differentiates multiple diseases (Step 2).

Step 1 can be carried out for example as follows.

For the bacteria contained in a saliva sample, the V1-V2 region of 16S rRNA is amplified by PCR, and nucleobase sequences of the fragments amplified by PCR are determined by using a next generation sequencer. Next, among the determined nucleobase sequences (reads), those reads that have passed quality checks are subjected to clustering according to 97% similarity to provide Operational Taxonomic Units (OTUs). 16S rRNA nucleobase sequences deposited in a genome database are referenced against the nucleobase sequences of the OTUs to identify bacteria type of each OTU. From the read count of each OTU, representation abundance of each bacteria type can be identified.

The step of acquiring representation abundances of multiple types of bacteria from an analysis of salivary microbiota for building a prediction model may also be done in the same way.

Step 2 may be carried out by determination unit 20 in the processor of the computer which inputs the acquired representation abundances of multiple types of bacteria into a prediction model to estimate risks of neurodegenerative diseases occurring in the test subject through stratification which differentiates multiple diseases.

The prediction model has been input, in advance, to the processor of the computer performing Step 2, and thus, as shown in Figure 3, when the representation abundances of multiple types of bacteria acquired in Step 1 are input into the prediction model, risks of neurodegenerative diseases occurring in the test subject can be estimated through stratification which differentiates multiple diseases, to output a prediction result.

### Examples

Below, the present invention will be described in further details by providing examples, but the present invention is not limited to these examples.

### Example 1 (stratification to differentiate HC, MCI, DE, and DLB)

In multiple cohorts, saliva (0.5 to 1.0 mL) was collected from patients who had been diagnosed as MCI, DE, or DLB based on cognitive function tests such as MMSE and HDS-R and brain image analyses (MRI/SPECT) as well as from healthy controls (HC). Since it has been suggested that microbiome may become different dependent on living environments and diet, the healthy controls were recruited from spouses or individuals living in the same households who had similar life habits, wherever possible. Furthermore, to minimize influence from drugs, patients taking antibiotics were excluded. From the saliva that had been collected, bacterial deoxyribonucleic acid (DNA) was extracted by using lysozyme and achromopeptidase. The extracted DNA was purified by using phenol/chloroform solutions etc. Using this DNA solution as a template, the V1-V2 region of the 16S rRNA gene was PCR-amplified by using the primers designed for the region. A next generation sequencer such as MiSeq was used to acquire nucleobase sequences from the obtained amplicons.

The acquired sequencing data underwent quality checks and removal of the primer sequences, and clustering according to 97% similarity was performed for an OTU analysis. 16S rRNA nucleobase sequences deposited in the genome database were referenced against the nucleobase sequences of the OTUs to perform identification of bacteria types, analysis of bacterial compositions, and the like. From the bacterial groups (at the phylum, genus, or species level) which represented 0.1% or more of the total read counts in each of HC, MCI, DE, and DLB groups, multiple bacteria types having high representation abundances and characteristic bacteria types (bacteria types showing significant differences to serve as markers) in each of the two-group pairs, namely HC and MCI, HC and DE, HC and DLB, MCI and DE, MCI and DLB, and DE and DLB, were taken and machine learning was performed to determine combinations of multiple bacteria which could provide stratification to differentiate these diseases at high accuracies.

A cohort, from a different population than the patient cohort used for building the model, was used to validate the prediction model built. Or, accuracy of the prediction was verified with the cohort used for building the model by cross-validation of the prediction model built.

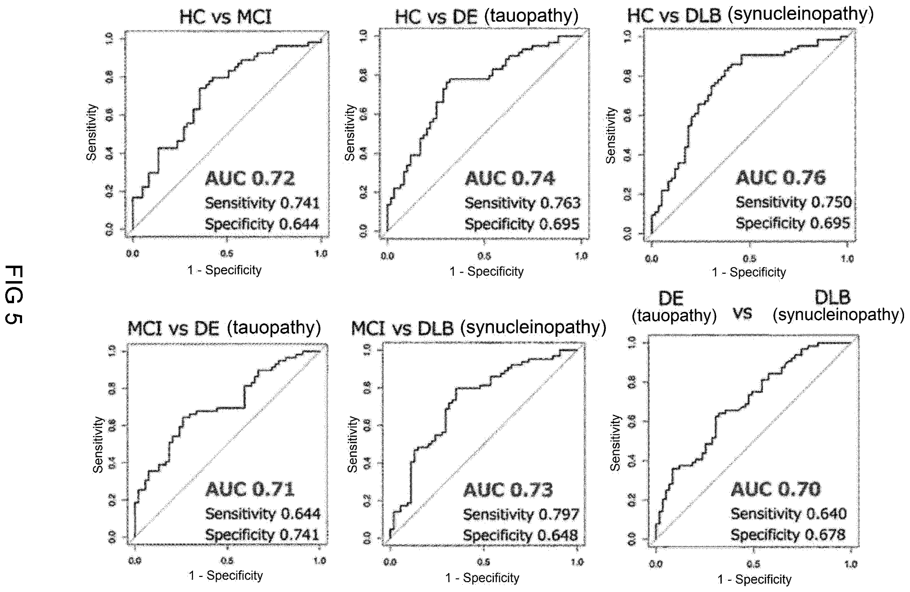

A prediction model was built by performing machine learning based on representation abundances of 94 bacteria types having high representation abundances at the species level (Table 1). The obtained AUC-RFs are shown in Figure 1. As is apparent in Figure 5, it became clear that neurodegenerative disease risks of HC and MCI, HC and DE (tauopathy), MCI and DE (synucleinopathy), MCI and DE (tauopathy), MCI and DLB (synucleinopathy), and DE (tauopathy) and DLB (synucleinopathy) could be predicted through stratification at high accuracies.

### Example 2 (stratification to differentiate HC, DLB, and PD)

Stratification to differentiate HC, DLB, and PD was attempted in a similar manner as Example 1. A prediction model was built by performing machine learning based on representation abundances of 95 bacteria types having high representation abundances at the species level (Table 2). The obtained AUC-RFs are shown in Figure 2.

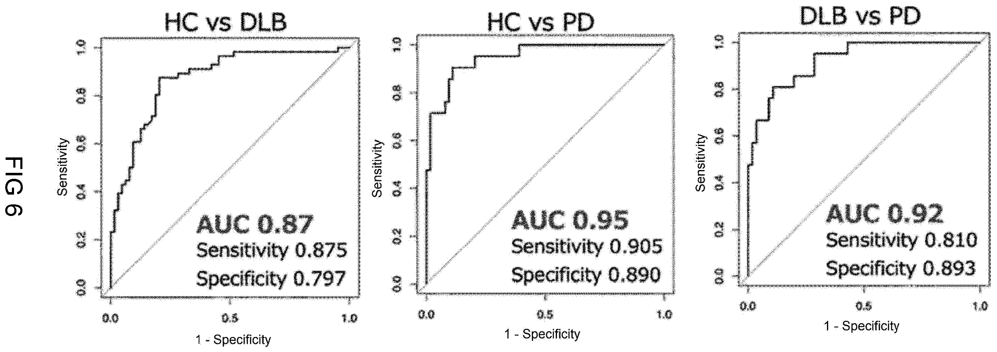

As is apparent in Figure 6, it became clear that neurodegenerative disease risks of HC, DLB, and PD could be predicted through stratification at high accuracies.

### Example 3 (stratification to differentiate HC, DLB, mild PD, and severe PD)

Stratification to differentiate HC, DLB, mild PD, and severe PD was attempted in a similar manner as Example 1.

A prediction model was built by performing machine learning based on representation abundances of 34 bacteria types having high representation abundances at the species level (Table 3). The obtained AUC-RFs are shown in Figure 3.

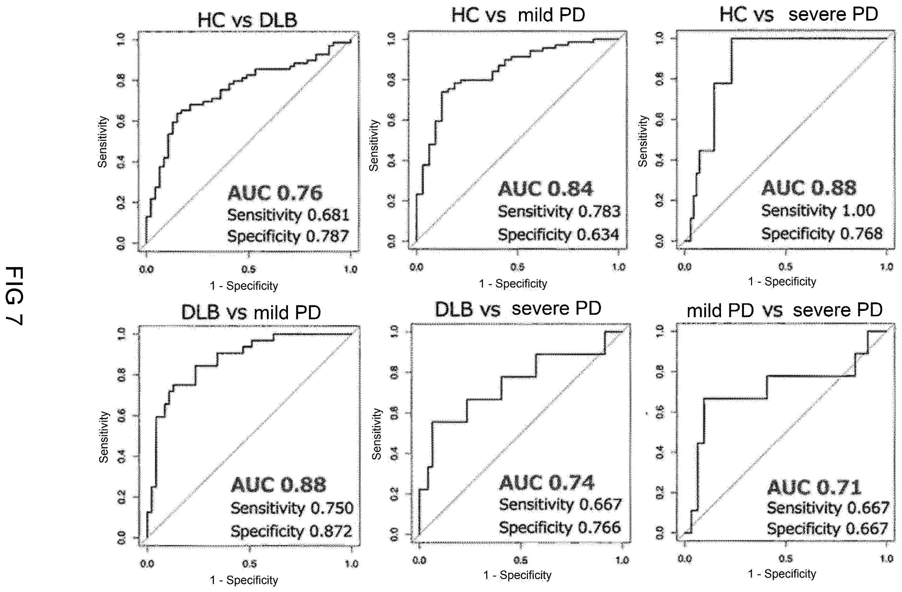

As is apparent in Figure 7, it became clear that neurodegenerative disease risks of HC, DLB, mild PD, and severe PD could be predicted through stratification at high accuracies.

### Example 4 (stratification to differentiate HC, DLB, early-stage PD, and late-stage PD)

Stratification to differentiate HC, DLB, early-stage PD, and late-stage PD was attempted in a similar manner as Example 1.

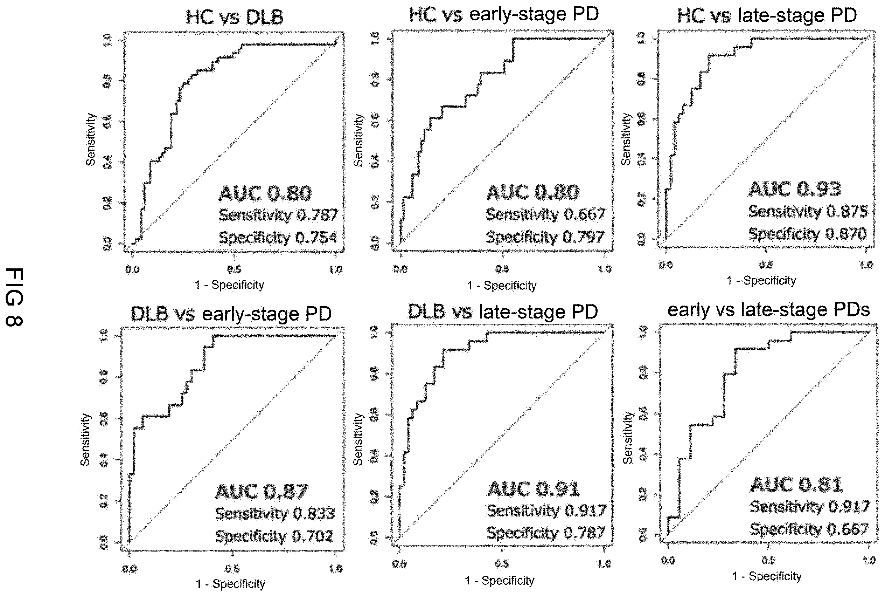

A prediction model was built by performing machine learning based on representation abundances of 74 bacteria types having high representation abundances at the species level (Table 4). The obtained AUC-RFs are shown in Figure 8.

As is apparent in Figure 8, it became clear that neurodegenerative disease risks of HC, DLB, early-stage PD, and late-stage PD could be predicted through stratification at high accuracies.

### Industrial Applicability

According to the methods and appratuses of the present invention, an easily obtainable, small amount of saliva sample can be used to perform accurate neurodegenerative disease risk determination through stratification to differentiate multiple diseases, such as healthy subjects, MCI and dementia, proteinopathy classes such as AD and DLB, and DLB and PD. Therefore, the present invention may be useful as a means for determining risks of neurodegenerative diseases in regular checkups and may contribute to early diagnosis of neurodegenerative diseases in a stratified fashion.

### Descriptions of the Symbols

100 Determination method
10 Acquisition unit
20 Determination unit
21 CPU
22 Storage unit
23 Control program
24 Prediction model
26 Input/output interface
200 Communication network

## Claims

1. A method of risk determination which estimates risks of neurodegenerative diseases through stratification which differentiates multiple diseases, the method executed by a processor of a computer, wherein the method comprises
a step of acquiring representation abundances of multiple types of bacteria from an analysis of salivary microbiota of a test subject; and
a step of inputting the acquired representation abundances of multiple types of bacteria into a prediction model to estimate risks of neurodegenerative diseases occurring in the test subject through stratification which differentiates multiple diseases,
wherein the prediction model has been generated by machine learning which entails obtaining an algorithm using, as explanatory variables, representation abundances of the multiple types of bacteria acquired from an analysis of salivary microbiota of healthy subjects and patients of multiple diseases belonging to the neurodegenerative diseases, and the disease states of the healthy subjects and the patients stratified across the multiple diseases belonging to neurodegenerative diseases, output as target variables, as training data,
wherein said multiple types of bacteria include bacteria types having high representation abundances in the analysis of the salivary microbiota of the patients stratified, and/or bacteria types showing significant differences in the representation abundances between patients of different diseases in the analysis of the salivary microbiota of the patients stratified.

2. The method of risk determination according to claim 1, wherein the stratification which differentiates multiple diseases comprises stratification to differentiate healthy aged subjects, mild cognitive impairment (MCI), and dementia (DE), stratification to differentiate healthy aged subjects, mild cognitive impairment (MCI), dementia (DE), and dementia with Lewy bodies (DLB), or stratification to differentiate Parkinson's disease (PD) and dementia with Lewy bodies (DLB).

3. The method of risk determination according to claim 1, wherein the multiple diseases include dementia with Lewy bodies (DLB) and Parkinson's disease (PD).

4. The method of risk determination according to any one of claims 1 to 3, wherein the multiple types of bacteria include one or more types of bacteria selected from the group consisting of [Eubacterium] brachy, Porphyromonas endodontalis, Alloprevotella tannerae, Capnocytophaga leadbetteri, Streptococcus gordonii, Campylobacter concisus, Tannerella forsythia, Filifactor alocis, [Eubacterium] nodatum, Streptococcus cristatus, Neisseria elongata, Treponema denticola, Actinomyces oris, [Eubacterium] saphenum, Streptococcus constellatus, Parvimonas micra, Prevotella denticola, Leptotrichia hofstadii, Fusobacterium nucleatum, Catonella morbi, Lactobacillus antri, Alloprevotella rava, Streptococcus anginosus, Prevotella jejuni, Streptococcus mitis, Gemella haemolysans, Neisseria macacae, Prevotella multiformis, Abiotrophia defectiva, Streptococcus salivarius, Streptococcus lactarius, Corynebacterium matruchotii, Oribacterium asaccharolyticum, Prevotella loescheii, Aggregatibacter segnis, Peptostreptococcus stomatis, Veillonella infantium, Capnocytophaga granulosa, Leptotrichia buccalis, Veillonella atypica, Streptococcus pseudopneumoniae, Corynebacterium durum, Granulicatella adiacens, [Eubacterium] sulci, Selenomonas infelix, Capnocytophaga sputigena, Lactobacillus crispatus, Streptococcus parasanguinis, Rothia mucilaginosa, Streptococcus sobrinus, Atopobium parvulum, Solobacterium moorei, Neisseria perflava, Bifidobacterium dentium, Actinomyces graevenitzii, Streptococcus mutans, Prevotella pallens, Porphyromonas gingivalis, Rothia dentocariosa, Fusobacterium periodonticum, Lactobacillus fermentum, Prevotella melaninogenica, Leptotrichia wadei, Lautropia mirabilis, Streptococcus infantis, Neisseria oralis, Prevotella pleuritidis, Prevotella oris, Lactobacillus paracasei, Lachnoanaerobaculum orale, Haemophilus parahaemolyticus, Prevotella nanceiensis, Lactobacillus salivarius, Streptococcus sanguinis, Haemophilus parainfluenzae, Lactobacillus vaginalis, Bacteroides heparinolyticus, Prevotella salivae, Gemella morbillorum, Gemella sanguinis, Prevotella shahii, Haemophilus haemolyticus, Schaalia odontolytica, Lactobacillus gasseri, Streptococcus australis, Streptococcus oralis, Prevotella histicola, Schaalia meyeri, Porphyromonas pasteri, Prevotella intermedia, Granulicatella elegans, Streptococcus downei, Parascardovia denticolens, Staphylococcus aureus, Haemophilus sputorum, and Prevotella oulorum.

5. An apparatus for risk determination which estimates risks of neurodegenerative diseases through stratification which differentiates multiple diseases, the apparatus comprising
a processor,
a storage unit which stores a computer program executed by the processor, and
a communication circuit which receives representation abundances of multiple types of bacteria from an analysis of microbiota of saliva obtained from a test subject;
wherein the processor executes the computer program to acquire the representation abundances of multiple types of bacteria received by the communication circuit and inputs the acquired representation abundances of multiple types of bacteria into a prediction model to estimate risks of neurodegenerative diseases occurring in the test subject through stratification which differentiates multiple diseases,
wherein the prediction model has been generated by machine learning which entails obtaining an algorithm using, as explanatory variables, representation abundances of the multiple types of bacteria acquired from an analysis of salivary microbiota of healthy subjects and patients of multiple diseases belonging to the neurodegenerative diseases, and the disease states of the healthy subjects and the patients stratified across the multiple diseases belonging to neurodegenerative diseases, output as target variables, as training data,
wherein said multiple types of bacteria include bacteria types having high representation abundances in the analysis of the salivary microbiota of the patients stratified, and/or bacteria types showing significant differences in the representation abundances between patients of different diseases in the analysis of the salivary microbiota of the patients stratified.

6. The apparatus for risk determination according to claim 5, wherein the stratification which differentiates multiple diseases comprises stratification to differentiate healthy aged subjects, mild cognitive impairment (MCI), and dementia (DE), stratification to differentiate healthy aged subjects, mild cognitive impairment (MCI), dementia (DE), and dementia with Lewy bodies (DLB), or stratification to differentiate Parkinson's disease (PD) and dementia with Lewy bodies (DLB).

7. The apparatus for risk determination according to claim 5, wherein the multiple diseases include dementia with Lewy bodies (DLB) and Parkinson's disease (PD).

8. The apparatus for risk determination according to any one of claims 5 to 7, wherein the multiple types of bacteria include one or more types of bacteria selected from the group consisting of [Eubacterium] brachy, Porphyromonas endodontalis, Alloprevotella tannerae, Capnocytophaga leadbetteri, Streptococcus gordonii, Campylobacter concisus, Tannerella forsythia, Filifactor alocis, [Eubacterium] nodatum, Streptococcus cristatus, Neisseria elongata, Treponema denticola, Actinomyces oris, [Eubacterium] saphenum, Streptococcus constellatus, Parvimonas micra, Prevotella denticola, Leptotrichia hofstadii, Fusobacterium nucleatum, Catonella morbi, Lactobacillus antri, Alloprevotella rava, Streptococcus anginosus, Prevotella jejuni, Streptococcus mitis, Gemella haemolysans, Neisseria macacae, Prevotella multiformis, Abiotrophia defectiva, Streptococcus salivarius, Streptococcus lactarius, Corynebacterium matruchotii, Oribacterium asaccharolyticum, Prevotella loescheii, Aggregatibacter segnis, Peptostreptococcus stomatis, Veillonella infantium, Capnocytophaga granulosa, Leptotrichia buccalis, Veillonella atypica, Streptococcus pseudopneumoniae, Corynebacterium durum, Granulicatella adiacens, [Eubacterium] sulci, Selenomonas infelix, Capnocytophaga sputigena, Lactobacillus crispatus, Streptococcus parasanguinis, Rothia mucilaginosa, Streptococcus sobrinus, Atopobium parvulum, Solobacterium moorei, Neisseria perflava, Bifidobacterium dentium, Actinomyces graevenitzii, Streptococcus mutans, Prevotella pallens, Porphyromonas gingivalis, Rothia dentocariosa, Fusobacterium periodonticum, Lactobacillus fermentum, Prevotella melaninogenica, Leptotrichia wadei, Lautropia mirabilis, Streptococcus infantis, Neisseria oralis, Prevotella pleuritidis, Prevotella oris, Lactobacillus paracasei, Lachnoanaerobaculum orale, Haemophilus parahaemolyticus, Prevotella nanceiensis, Lactobacillus salivarius, Streptococcus sanguinis, Haemophilus parainfluenzae, Lactobacillus vaginalis, Bacteroides heparinolyticus, Prevotella salivae, Gemella morbillorum, Gemella sanguinis, Prevotella shahii, Haemophilus haemolyticus, Schaalia odontolytica, Lactobacillus gasseri, Streptococcus australis, Streptococcus oralis, Prevotella histicola, Schaalia meyeri, Porphyromonas pasteri, Prevotella intermedia, Granulicatella elegans, Streptococcus downei, Parascardovia denticolens, Staphylococcus aureus, Haemophilus sputorum, and Prevotella oulorum.
